# EUROPEAN PATENT APPLICATION

(11) **EP 1 593 429 A1**
(43) Date of publication of application: **09.11.2005**
(21) Application number: 04380103.4
(22) Date of filing: 05.05.2004
(51) Int. Cl.: B01J 29/064, B01J 29/74, C10G 45/64, C07C 5/27

(54) **Process for obtaining a light paraffin isomerisation catalyst**

(71) Applicant: REPSOL YPF S.A., 28046 Madrid (ES)
(72) Inventor: Jimenez Sinchidrian, César, 14004 Cordoba (ES); Romero Salguero, Francisco José, 14005 Cordoba (ES); Roldan Mesa, Rafael, 14007 Cordoba (ES); Lacalle Fernandez, David, 28003 Madrid (ES); Gomez Martin, Juan Pedro, 28916 Leganes (ES)
(74) Representative: Davila Baz, Angel

(57) **Abstract**

A process for obtaining a light paraffin isomerisation catalyst, comprising the steps of treating a support comprising a zeolite with a chelating agent, calcination, deposition of a metal or a mixture of metals of group VIII on the support, and calcination; the catalyst obtainable by said process and an isomerisation process for C4 to C9 hydrocarbons involving the use of said catalyst.
The chelating agent is preferably selected from dimethylglyoxime, cyclohexanedione dioxime, hydroxylamine, cyclohexane diamine, n-hexanedione dioxime, phenanthrenquinone dioxime, N,N-dimethylformamide, urea, oxamide and other oximes, dioximes and N-containing organic compounds. The zeolite is zeolite beta, mordenite, faujasite, ferrierite or ZSM-5.

## Description

The present invention refers to a process for obtaining a new catalyst for isomerisation of light paraffins based on synthetic zeolite pre-treated with a chelating agent. The pre-treated zeolite mixed with a refractory oxide material and, at least, one metal of the group VIII is calcined.

### PRIOR ART

The isomerisation reaction of light paraffins is of considerable importance in the petroleum industry. The evolution of gasoline specifications (unleaded gasoline, lower aromatic content) makes the presence of isomerised paraffins in the gasoline formulation become more important.

Over the years different types of catalysts have been developed for the isomerisation of light paraffins [see M. Belloum, C. Travers, and J. P. Bournonville, REV. INST. FR. PÉTROL. Vol. 46, p. 89 (1991)]. Chronologically they can be classified as:
- First generation catalysts: AlCl₃ and other strong Lewis acids based catalysts
- Second generation catalysts: Pt/Al₂O₃ catalysts
- Third generation catalysts: Pt/Al₂O₃-HF, Pt/Al₂O₃-HCl catalysts
- Fourth generation catalysts: Zeolite based catalysts such as Pt/zeolite, Pd/zeolite
- Fifth generation catalysts: Sulfated zirconia catalysts

There is a wide range of applications of these catalysts in the isomerisation of paraffins. Some of the catalysts are easily synthesized, which means low cost catalysts. Other catalysts, with higher costs, present better results. In all cases efforts have been made to improve the catalysts synthesis to provide better stability and activity.

There has been an effort to improve the fourth generation catalysts by optimizing the metal dispersion or treating the support in order to modify its activity (U.S. Pat. No. 5,095,169). Some of these processes treat the support (zeolite) by dealumination to obtain an optimal acidity. These treatments are generally based in washing the zeolite in an acid mineral medium such as HNO₃, H₃PO₄, HCl, etc., or in an organic acid medium (oxalic acid, phtalic acid, etc).

Most of these catalysts give good isomerisation results for light paraffins in the range of C4-C5-C6. Nevertheless, for the isomerisation of heavier paraffins (C7, C8) the results become poorer because of the high amount of cracking reactions.

### DESCRIPTION OF THE INVENTION

A process for obtaining new light paraffin isomerisation catalysts, based on group VIII metals, added to a zeolite, pre-treated with a chelating agent, has now been found. These catalysts have shown to have an excellent activity and selectivity in the isomerisation of light paraffins in the range of C4 to C9. Additionally, they present an excellent conversion and selectivity to mono and di-branched isoparaffins, while cracking reactions are minimized. Isomerising hydrocarbons in the presence of hydrogen by contacting said hydrocarbons with these new catalysts provides a product with a high octane number and a low production of gases coming from cracking reactions.

Accordingly, the present invention is related, according to a first aspect, to a process for obtaining a light paraffin isomerisation catalyst. Said process comprises the following steps:
a) treatment of a support comprising zeolite, with a chelating agent, at a temperature between 10 and 200°C, to a final ratio of chelating agent/zeolite (w/w) between 0.01 and 10;
b) calcination at temperatures between 200 and 700 °C;
c) deposition of a metal or a mixture of metals of group VIII on the support, in an amount between 0.01 and 5 wt.%, referred to the total amount of catalyst;
d) calcination at temperatures between 200 and 700°C.

According to a preferred embodiment, the catalyst obtained by anyone of steps b) to d) may be mixed with a refractory metal oxide. Preferably, the zeolite/oxide ratio is between 1 and 9.

The starting zeolite can be natural or synthetic; preferably, the zeolite is selected from beta zeolite, mordenite, faujasite, ferrierite or ZSM-5.

Preferably, the zeolite has a SiO₂/Al₂O₃ ratio between 2 and 150; more preferably, the zeolite is a beta zeolite with a SiO₂/Al₂O₃ ratio between 20 and 150. Even more preferably the SiO₂/Al₂O₃ ratio is about 25.

The support of step a) may either exclusively consist of zeolite, or it may comprise both a zeolite and a refractory metal oxide.

The refractory metal oxide that may initially form part of the support or that may be mixed with the zeolite support after either steps b), c) or d), as indicated before, is preferably selected from alumina, bentonite and sepiolite.

The chelating agent is preferably selected from the group consisting of dimethylglyoxime, diphenylglyoxime, cyclohexanedione dioxime, hydroxylamine, cyclohexane diamine, n-hexanedione dioxime, phenanthrenquinone dioxime, N,N-dimethylformamide, urea, oxamide and other oximes, dioximes and N-containing organic compounds. Dimethylglyoxime is the preferred agent in this invention. It may be added alone or in a solution. The solvent can be water or organic compounds, such as ketones, alcohols or ethers, alone or mixed with water or an acid solution (phosphoric acid solution, nitric acid solution). A non-acid solution is preferred. The treatment temperature is between 10 and 200 °C, and the zeolite is contacted with the chelating agent or the chelating agent solution, and the contact time will be between 2 to 24 hours, preferably about 12 hours. During the contact between zeolite and the chelating agent, reflux can be used. In fact, it is preferable to use reflux. The obtained solid is then filtered and dried at about 80°C during about 12 hours.

According to another preferred embodiment, the ratio of chelating agent to zeolite is between 0.1 and 0.5.
Step a) is preferably performed at about 120°C, during about 12 hours.
Step b) is preferably performed at about 580°C.
Step d) is preferably performed at about 400°C.

The metal or mixture of metals of group VIII is preferably Pt, Pd or a mixture of both. More preferably the metal added is Pt. The metal or mixture thereof is added to the treated zeolite alone or in a mixture of the treated zeolite with the inorganic refractory oxide. The amount of metal is between 0.01 to 5% by weight referred to the total weight of the catalyst. The preferred amount is about 0.5 wt.%.

The metal can be deposited on the catalyst in any suitable way, e.g. by impregnation, ion-exchange, precipitation or co-mulling.

According to another aspect, the present invention is related to a light paraffin isomerisation catalyst obtainable by the process according to the present invention.

An additional aspect of the present invention is a light paraffin isomerisation process that comprises the use of said isomerisation catalyst obtainable by the process according to the present invention. Preferably, said light paraffin isomerisation process is carried out at a temperature between 150 and 400°C, preferably between 225 and 275°C, and at a pressure between 1 and 100 bar, preferably between 10 and 50 bar, and space velocities of 0.5 to 8 h⁻¹. The molar hydrogen/hydrocarbon ratio is preferably between 0.1 and 10. According to a preferred embodiment, C₄-C₉ hydrocarbons are subjected to the isomerisation process. According to another preferred embodiment, the catalyst is reduced in hydrogen atmosphere prior to the isomerisation process. Said reduction is preferably performed at temperatures between 100 and 600°C.

Further, according to a last aspect, the present invention is related to isomerised hydrocarbons or mixtures whenever obtained by the light paraffin isomerisation catalyst according to the present invention.

In the following, the present invention is further illustrated by means of a series of non-restrictive examples.

### EXAMPLES

### Examples of zeolite pre-treatment.

### Example 1

100 grammes of a commercially available beta zeolite were stabilized at 580°C during three hours. After cooling, the beta zeolite is introduced in a 1000 ml flask containing 480 ml of distilled water with 14 grammes of dimethylglyoxime suspended. The flask is heated up smoothly to the reflux temperature and once the temperature is reached it is maintained during 14 hours. After this time the flask is cooled and then the suspension is filtered in a Buchner. The solid product is washed three times with 50 ml of hot water each time. The solid obtained by this procedure is dried and calcined at 580°C during three hours. The zeolite thus obtained is hereinafter called **zeo-DMG**.

### Example 2

The experiment described in example 1 was repeated but using, instead of dimethylglyoxime, its sodium salt. The zeolite thus obtained is hereinafter called zeo-**DMG-Na**

### Example 3

100 grammes of a commercially available beta zeolite were stabilized at 580°C during three hours. After cooling, the beta zeolite is introduced in a 1000 ml flask containing 480 ml of acid solution 0.25 M (using HNO₃ as acid) with 14 grammes of dimethylglyoxime suspended. The flask is heated up smoothly to the reflux temperature and once the temperature is reached it is maintained during 14 hours. After this time the flask is cooled and then the suspension is filtered in a Buchner. The solid product is washed three times with 50 ml of hot water each time. The solid obtained by this procedure is dried and calcined at 580°C during three hours. The zeolite thus obtained is hereinafter called **zeo-DMG-H.**

### Example 4

The experiment described in example 1 was repeated but using H₃PO₄, instead of HNO₃. The zeolite thus obtained is hereinafter called **zeo-DMG-H3**

### Examples of metal deposition

### Example 5

20 grammes of zeo-DMG are introduced in the flask of a rotavapor containing 102.5 milliliters of an aqueous solution 0.005 M of tetrammineplatinum (II) nitrate in order to obtain an amount of metal deposited on the catalyst of 0.5 wt.% referred to the total weight of catalyst. After stirring for 60 minutes, the vacuum system is connected (15 torr) and the solvent is removed at 50°C. The catalyst thus obtained is dried during 24 hours. The catalyst is loaded in a reactor and it is activated. The catalyst thus obtained is hereinafter called **Pt-zeo-DMG**

### Example 6

The experiment described in example 5 was repeated but using zeo-DMG-Na. The catalyst thus obtained is hereinafter called **Pt-zeo-DMG-Na**

### Example 7

The experiment described in example 5 was repeated but using zeo-DMG-H. The catalyst thus obtained is hereinafter called **Pt-zeo-DMG-H**

### Example 8

The experiment described in example 5 was repeated but using zeo-DMG-H3. The catalyst thus obtained is hereinafter called **Pt-zeo-DMG-H3**

### Example 9

The experiment described in example 5 was repeated but using 188 milliliters of an aqueous solution 0.005 M of tetramminepalladium (II) nitrate in order to obtain an amount of palladium of 0.5 wt.% referred to the total weight of the catalyst. The catalyst thus obtained is hereinafter called **Pd-zeo-DMG**

### Example 10

The experiment described in example 5 was repeated but using 145 milliliters of an aqueous solution 0.005 M of tetrammineplatinum (II) nitrate and 0.005M of tetramminepalladium (II) nitrate in order to obtain an amount of total metal of 1 wt.% referred to the total weight of the catalyst. The catalyst thus obtained is hereinafter called **Pt-Pd-zeo-DMG**

### Example 11

The experiment described in example 5 was repeated, but the Pt-zeo-DMG catalyst is placed in a U-tube where it is reduced with hydrogen (20 mL min⁻¹) at 400°C during 1 hour. After that step, the catalyst is submitted to a new metal deposition, contacting the catalyst with 188 milliliters of an aqueous solution 0.005 M of tetramminepalladium (II) nitrate in order to obtain a total amount of metal of 1 wt.% referred to the total weight of the catalyst. After stirring for 60 minutes, the vacuum system is connected (15 torr) and the solvent is evaporated at 50°C. The catalyst thus obtained is dried during 24 hours. The catalyst is loaded in a reactor and it is activated. The catalyst thus obtained is hereinafter called **Pt-Pd-1-zeo-DMG**

### Example 12

The experiment described in example 6 was repeated, but the Pt-zeo-DMG catalyst, is placed in a U-tube where it is reduced with hydrogen (20 mL min⁻¹) at 400°C during 1 hour. After that step, the catalyst is submitted to a new metal deposition, contacting the catalyst with 102.5 milliliters of an aqueous solution 0.005 M of tetraamineplatinum (II) nitrate in order to obtain a total amount of metal of 1% by weight referred to the total weight of the catalyst. After stirring for 60 minutes, the vacuum system is connected (15 torr) and the solvent is evaporated at 50°C. The catalyst thus obtained is dried during 24 hours. The catalyst is loaded in a reactor and it is activated. The catalyst thus obtained is hereinafter called **Pt-Pd-2-zeo-DMG**

### Examples of catalytic activity in isomerisation

### Example 13

The catalysts prepared in the examples 5, 6, 7, and 8 have been tested with a feed composed by 65 wt.% n-hexane (n-C6), 20 wt.% cyclohexane (cC6) and 15 wt.% n-heptane (n-C7). The reactions have been carried out in a fixed bed reactor at atmospheric pressure. The reactor outlet was connected to a gas chromatograph to analyze the reaction products.

The first step is the activation of catalysts. Once the catalyst (one gramme) is loaded in the reactor, it is established an oxygen flow of 20 milliliters per minute during one hour at 400°C. After this the catalyst is passivated at 400°C during 15 minutes with a nitrogen flow of 20 milliliters per minute. Then the catalyst is reduced in a hydrogen flow of 20 milliliters per minute at 400°C during 1 hour. The catalyst is then ready for the isomerisation of paraffins.

The conditions used are the following:
Hydrogen flow: 60 milliliters per minute.
Catalyst bed temperature: between 225 and 350°C, better 250°C.
Hydrocarbon feed flow: between 2.7 and 5.4 milliliters per hour.
Hydrogen/feed molar ratio: between 1 and 8, better 4.
Weight hourly space velocity (WHSV): between 1 and 6 h⁻¹, better 3.7 h⁻¹.

The results obtained are presented in the following tables (Conversion is measured by the ratio between the total amount of converted normal paraffins and normal paraffins in the feed):

| | EXAMPLE 5 | EXAMPLE 6 | EXAMPLE 7 | EXAMPLE 8 |
|---|---|---|---|---|
| Catalyst | Pt-zeo-DMG | Pt-zeo-DMG-Na | Pt-zeo-DMG-H | Pt-zeo-DMG-H3 |
| nC6 Conversion | 37.1% | 29.8% | 40.3% | 35.5% |
| cC6 Conversion | 63.2% | 55.8% | 64.6% | 60.2% |
| nC7 Conversion | 78.8% | 70.0% | 80.3% | 77.5% |
| **Total conversion** | **49.1%** | **41.5%** | **51.6%** | **47.2%** |
| Cracking | 3.5% | 4.2% | 4.0% | 4.1% |
| Cracking is the yield of cracked products (C1-C4) | | | | |

## Claims

1. A process for obtaining a light paraffin isomerisation catalyst, **characterized in that** it comprises the following steps:
a) treatment of a support comprising a zeolite, with a chelating agent, at a temperature between 10 and 200°C, to a final ratio of chelating agent/zeolite (w/w) between 0.01 and 10;
b) calcination at temperatures between 200 and 700 °C;
c) deposition of a metal or a mixture of metals of group VIII on the support, in an amount between 0.01 and 5 wt.%, referred to the total amount of catalyst;
d) calcination at temperatures between 200 and 700°C.

2. Process according to claim 1, wherein the support obtained by anyone of steps b) to d) is mixed with a refractory metal oxide.

3. Process according to anyone of claims 1 to 2, wherein the zeolite is beta zeolite.

4. Process according to anyone of claims 1 to 2, wherein the zeolite is mordenite, faujasite, ferrierite or ZSM-5.

5. Process according to anyone of claims 1 to 4, wherein the support comprises a zeolite and a refractory metal oxide.

6. Process according to anyone of claims 2 and 5, wherein the refractory metal oxide is alumina, bentonite or sepiolite.

7. Process according to anyone of claims 2, 5 and 6, wherein the zeolite/oxide ratio is between 1 and 9.

8. Process according to anyone of claim 1 to 7, wherein the chelating agent is dimethylglyoxime.

9. Process according to anyone of claims 1 to 7, wherein the chelating agent is selected from the group consisting of diphenylglyoxime, cyclohexanedione dioxime, hydroxylamine, cyclohexane diamine, n-hexanedione dioxime, phenanthrenquinone dioxime, N,N-dimethylformamide, urea, oxamide and other oximes, dioximes and N-containing organic compounds.

10. Process according to anyone of claims 1 to 9, wherein the metal or mixture of metals of group VIII is at least one of Pt and Pd.

11. Process according to anyone of claims 1 to 10, wherein the zeolite has a SiO₂/Al₂O₃ ratio between 2 and 150.

12. Process according to anyone of claims 1 to 11, wherein the chelating agent/zeolite ratio is between 0.1 and 0.5.

13. A light paraffin isomerisation catalyst obtainable by the process of claims 1 to 12.

14. A light paraffin isomerisation process comprising the use of the isomerisation catalyst of claim 13.

15. A light paraffin isomerisation process according to claim 14, wherein the isomerisation is carried out at a temperature between 150 and 400°C and a pressure between 1 and 100 bar.

16. A light paraffin isomerisation process according to any one of claims 14 and 15, wherein C₄-C₉ hydrocarbons are subjected to isomerisation.

17. A light paraffin isomerisation process according to anyone of claims 14 to 16 wherein the catalyst is reduced in hydrogen atmosphere prior to the isomerisation process.

18. Isomerised hydrocarbons or mixtures thereof obtainable by the process of claims 14 to 17.
